# EUROPÄISCHE PATENTSCHRIFT

(11) **EP 3 268 091 B1**
(45) Veröffentlichungstag und Bekanntmachung des Hinweises auf die Patenterteilung: **06.03.2019**
(21) Anmeldenummer: 16701523.9
(22) Anmeldetag: 26.01.2016
(51) Int. Cl.: A61Q 5/06, A61K 8/81

(54) **MITTEL UND VERFAHREN ZUR TEMPORÄREN VERFORMUNG KERATINHALTIGER FASERN**
PRODUCT AND METHOD FOR THE TEMPORARY SHAPING OF KERATIN-CONTAINING FIBERS
PRODUIT ET PROCÉDÉ DE MISE EN EN FORME TEMPORAIRE DE FIBRES KÉRATINIQUES

(30) Priorität: 09.03.2015 DE 102015204153
(43) Veröffentlichungstag der Anmeldung: 17.01.2018
(73) Patentinhaber: Henkel AG & Co. KGaA, 40589 Düsseldorf (DE)
(72) Erfinder: LANGE, Julia Bibiane, 24576 Bad Bramstedt (DE); PULS, Anna, 21423 Winsen (Luhe) (DE); MARTINEZ, Cyrielle, 22765 Hamburg (DE); RICHTERS, Bernd, 21129 Hamburg (DE)
(86) Internationale Anmeldenummer: PCT/EP2016/051565
(87) Internationale Veröffentlichungsnummer: WO 2016/142091

(56) Entgegenhaltungen:
- EP-A2- 1 878 423
- WO-A1-2015/095870
- US-A1- 2006 251 600
- US-B1- 6 482 394
- DATABASE GNPD [Online] MINTEL; 1. Dezember 2014 (2014-12-01), "Styling Mousse", XP002754994, Database accession no. 2832599
- DATABASE GNPD [Online] MINTEL; 1. März 2014 (2014-03-01), "Ultra Power Styling Gel", XP002754995, Database accession no. 2324538
- DATABASE GNPD [Online] MINTEL; 1. November 2013 (2013-11-01), "Full Form Mousse", XP002754996, Database accession no. 2237163
- DATABASE GNPD [Online] MINTEL; 1. Juni 2014 (2014-06-01), "Curl Enhancing Styling Foam", XP002754997, Database accession no. 2480107
- DATABASE GNPD [Online] MINTEL; 1. September 2014 (2014-09-01), "Extreme Max Gel for Men", XP002754998, Database accession no. 2683391
- DATABASE GNPD [Online] MINTEL; 1. Juli 2004 (2004-07-01), "Wellaflex Mousse", XP002754999, Database accession no. 284667
- ANONYMOUS: "Ashland brings performance and style to crystal clear gel with AquaStyle", INTERNET CITATION, April 2014 (2014-04), Seiten 1-2, XP002751471, Gefunden im Internet: URL:http://files.shareholder.com/downloads /ASH/0x0x739330/f61f8ac2-932b-4e99-afa7-0d 1962337028/ASH_News_2014_4_1_Products_and_ Services.pdf [gefunden am 2015-11-26]
- Anonymous: "Ashland brings performance and style to crystal clear gel with AquaStyle(TM) (NYSE:ASH)", , 1. April 2014 (2014-04-01), XP055205095, Gefunden im Internet: URL:http://investor.ashland.com/releasedet ail.cfm?releaseid=836949 [gefunden am 2015-07-28]

## Beschreibung

Die vorliegende Erfindung betrifft eine kosmetische Zusammensetzung zur Haarfestigung bzw. zur temporären Umformung von keratinischen Fasern, insbesondere menschlichen Haaren, wobei die Zusammensetzung eine Kombination eines kationischen und eines anionischen Polymers enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden. Handelt es sich bei dem Stylingmittel um ein Gel oder eine Paste, sollen die Polymere zudem verdickende Eigenschaften besitzen.

So beschreibt die deutsche Patentanmeldung DE 10 2011 089 170 A1 Haarkosmetika zur temporären Haarverformung, die neben weiteren Bestandteilen auch mindestens eine polymere quartäre Ammoniumverbindung aus der Gruppe der Vinylpyrrolidon Copolymere enthalten.

US6482394 B1 offenbart in Beispiele 11 und 12 ein Haarstylingmittel enthaltend Luviquat Hold (polyquaternium 46) und Luvimer 36D (Methacrylsäure/t-Butylacrylat/Ethylacrylat Copolymer)

Weiterhin sind hydrophob modifizierte Acrylatcopolymere (INCI: Acrylates Copolymer (and) Water) im Handel erhältlich, die im Wesentlichen als Verdickungsmittel wirken. Das Datenblatt AquaStyle® SH-100 Polymer (Ashland Inc.) beschreibt ein solches Acrylatcopolymer und dessen Verwendung in Kombination mit Carbomeren. Es wird eine Eignung für kristallklare Haargele, eine gute Anfangssteifheit, Feuchtigkeitsbeständigkeit und eine Langzeitwirkung beschrieben.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen. Insbesondere sind derzeit erhältliche Stylingmittel noch dahingehend verbesserbar, dass eine gute Kombination aus Steifheit (Stiffness) und Langzeithalt (High Humidity Curl Retention) nicht immer ausreichend gewährleistet wird. Es ist daher eine Aufgabe der vorliegenden Erfindung, derartige Stylingmittel bereitzustellen, die neben den oben genannten Eigenschaften insbesondere sowohl eine gute Steifheit als auch einen guten Langzeithalt ergeben.

Dies wurde erfindungsgemäß durch eine Kombination zweier spezifischer Polymere erreicht.

Durch die vorliegende Erfindung wird bereitgestellt:
Eine kosmetische Zusammensetzung zur temporären Umformung keratinischer Fasern, welche enthält:
(a) mindestens eine polymere quartäre Ammoniumverbindung aus der Gruppe der Vinylpyrrolidon Copolymere, welche zumindest aus den folgenden Monomereinheiten aufgebaut ist:
   (a1) Vinylpyrrolidon
   (a2) Methacrylamidopropyltrimethylammoniumchlorid
   und
(b) mindestens ein anionisches Acrylatcopolymer (b), welches zumindest aus folgenden Monomereinheiten aufgebaut ist:
   (b1) mindestens eine (Meth)Acrylsäureeinheit
   (b2) mindestens eine (Meth)Acrylsäureethylestereinheit
   (b3) mindestens eine (Meth)Acrylsäureestereinheit, die von der (Meth)Acrylsäureethylestereinheit (b2) verschieden ist und eine hydrophobe Gruppe als Estergruppe aufweist.

Bevorzugt ist eine kosmetische Zusammensetzung wobei das Vinylpyrrolidon-Copolymer (a) eines mit der INCI-Bezeichnung Polyquaternium-28, insbesondere Gafquat® HS 100 (ISP), ist.

Bevorzugt ist eine kosmetische Zusammensetzung, wobei das anionische Acrylatcopolymer (b) eines mit der INCI-Bezeichnung Acrylates Copolyme (and) Water, insbesondere AquaStyle SH-100 (Ashland Inc.), ist.

Weiter bevorzugt ist eine kosmetische Zusammensetzung, wobei das Vinylpyrrolidon-Copolymer (a) Gafquat® HS 100 (ISP) ist und das anionische Acrylatcopolymer (b) AquaStyle® SH-100 (Ashland Inc.) ist.

Es wurde im Rahmen der vorliegenden Erfindung überraschend festgestellt, dass durch Kombination zweier an sich bekannter Bestandteile, die in Stylingprodukten bereits Anwendung finden, eine verbesserte Feuchtebeständigkeit von Stylingprodukten erhalten werden kann. Andere üblicherweise geforderte Eigenschaften von Stylingprodukten wie Langzeithalt, Steifheit und geringe Klebrigkeit blieben dabei erhalten. Eine derartige gute Kombination von Eigenschaften war selbst bei Kenntnis der Einzelkomponenten nicht zu erwarten und war überraschend. Es zeigte sich experimentell, dass durch die Kombination der beiden Komponenten ein stark überadditiver, also synergistischer Effekt hinsichtlich der Feuchtebeständigkeit erhalten wurde, was sich im HHRC-Test (High Humidity Curl Retention-Test) manifestierte.

Der Begriff keratinische Fasern umfasst erfindungsgemäß Pelze, Wolle und Federn, insbesondere aber menschliche Haare.

Die wesentlichen Bestandteile der erfindungsgemäßen kosmetischen Zusammensetzung sind das Vinylpyrrolidon-Copolymer (a) und das von dem Copolymer (a) verschiedene anionische Acrylatcopolymer (b).

Die erfindungsgemäßen Zusammensetzungen enthalten als ersten wesentlichen Bestandteil eine polymere quartäre Ammoniumverbindung aus der Gruppe der Vinylpyrrolidon Copolymere (a). Kosmetische Zusammensetzungen mit einem auf das Gesamtgewicht der Zusammensetzung bezogenen Gewichtsanteil des Copolymers a) von 1,0 bis 5,0 Gew.-%, vorzugsweise 1,5 bis 4,0 Gew.-% und insbesondere 2,0 bis 3,0 Gew.-% zeichnen sich durch besonders vorteilhafte kosmetische Eigenschaften aus.

Ein besonders geeignetes Copolymer (a) wird durch Umsetzung von Vinylpyrrolidon mit Methacrylamidopropyltrimethylammoniumchlorid erhalten.

Diese Copolymere von Vinylpyrrolidon mit Methacrylamidopropyltrimethylammoniumchlorid (MAPTAC) lassen sich durch die allgemeine Formel 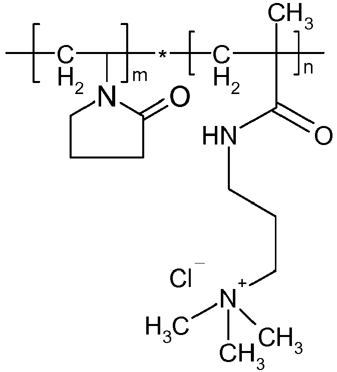 beschreiben, wobei die Indices m und n je nach Molmasse des Polymers variieren und nicht bedeuten sollen, dass es sich um Blockcopolymere handelt. Vielmehr können Struktureinheiten im Molekül statistisch verteilt vorliegen.

Besonders bevorzugte kosmetische Mittel sind dadurch gekennzeichnet, dass sie als Vinylpyrrolidon-Copolymer (a) Copolymere von Methacrylamidopropyltrimethylammoniumchlorid (MAPTAC) mit Vinylpyrrolidon enthalten, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

Besonders bevorzugte kosmetische Mittel sind weiter dadurch gekennzeichnet, dass die Copolymere (a) Molmassen von 10 bis 1000 kDa, vorzugsweise von 25 bis 900 kDa, weiter bevorzugt von 50 bis 800 kDa und insbesondere von 100 bis 750 kDa, aufweisen.

Ein ganz besonders bevorzugtes Copolymer (a) wird nach der INCI-Nomenklatur als Polyquaternium-28 bezeichnet. Ein solches Polymer wird beispielsweise unter der Handelsbezeichnung Gafquat® HS-100 (CAS Nummer: 131954-48-8) von der Firma ISP vertrieben.

Die erfindungsgemäßen kosmetischen Zusammensetzungen enthalten als zweiten wesentlichen Bestandteil ein anionisches Acrylatcopolymer (b).

Das anionische Acrylatcopolymer (b) ist zumindest aus folgenden Monomereinheiten aufgebaut: mindestens einer (Meth)Acrylsäureeinheit (b1), mindestens einer (Meth)Acrylsäureethylestereinheit (b2) und mindestens einer (Meth)Acrylsäureestereinheit (b3), die von der (Meth)Acrylsäureethylestereinheit (b2) verschieden ist und eine hydrophobe Gruppe als Estergruppe aufweist.

Das Copolymer (b) kann erfindungsgemäß aus weiteren Monomereinheiten aufgebaut sein. Gemäß einer bevorzugten Ausführungsform der Erfindung ist das Copolymer (b) aber nur aus den Einheiten (b1), (b2) und (b3) aufgebaut, d. h. es besteht aus von diesen Monomereinheiten abgeleiteten Einheiten.

Die mindestens eine (Meth)Acrylsäureeinheit (b1) kann eine Methacrylsäure- oder Acrylsäureeinheit sein, wobei eine Methacrylsäureeinheit bevorzugt ist.

Die mindestens eine (Meth)Acrylsäureethylestereinheit (b2) kann eine Methacrylsäureethylestereinheit oder eine Acrylsäureethylestereinheit, wobei eine Acrylsäureethylestereinheit bevorzugt ist.

Die mindestens eine (Meth)Acrylsäureestereinheit (b3) kann erfindungsgemäß eine (Meth)Acrylsäurealkylestereinheit sein. Die Alkylgruppe der (Meth)Acrylsäurealkylestereinheit dient zur Steuerung der Hydrophobizität des Copolymers. Die Alkylgruppe ist bevorzugt eine lineare oder verzweigte Alkylgruppe mit 2 bis 30 Kohlenstoffatomen, bevorzugter 3 bis 12 Kohlenstoffatomen. Die hydrophobe Gruppe kann erfindungsgemäß auch eine andere hydrophobe als eine Alkylgruppe sein, z. B. eine aromatische Kohlenwasserstoffestergruppe. Als Beispiel sei eine substituierte oder unsubstituierte Phenylestergruppe oder substituierte oder unsubstituierte Alkylenphenylestergruppe genannt, z. B. eine Benzylestergruppe.

Die Viskosität des in der kosmetischen Zusammensetzung verwendeten anionischen Acrylatcopolymers (b) bei einem Feststoffgehalt von 2 Gew.-% und neutralisierter Lösung bei 25°C ist bevorzugt höchstens 60000 bis 120000 cPS.

Geeignete anionische Acrylatcopolymere (b) sind im Handel unter der INCI-Bezeichnung Acrylates Copolymer (and) Water erhältlich. Am bevorzugtesten ist das anionische Acrylatcopolymer (b) AquaStyle® SH-100 Polymer von Ashland, Inc. Dieses hat in der im Handel erhältlichen Form einen Feststoffgehalt von etwa 28 bis 32 Gew.-% und einen pH-Wert von 2,1 bis 4,0.

Die kosmetische Zusammensetzung der vorliegenden Erfindung enthält das Vinylpyrrolidon-Copolymer (a) und Acrylatcopolymer (b) in für Stylingmittel üblichen und geeigneten Mengen, die für die spezielle Anwendung und Konfektionierung angepasst werden können.

Die erfindungsgemäße Zusammensetzung kann das Vinylpyrrolidon-Copolymer (a) beispielsweise in einer Menge von 1,0 bis 5,0 Gew.-%, bezogen auf das Gesamtgewicht der erfindungsgemäßen Zusammensetzung, enthalten. Bevorzugter sind Anteile des Vinylpyrrolidon-Copolymer (a) von 1,5 bis 4,0 Gew.-% und insbesondere von 2,0 bis 3,0 Gew.-%, jeweils angegeben als Feststoffgehalt aktiver Substanz in der kosmetischen Zusammensetzung.

Die erfindungsgemäße kosmetische Zusammensetzung enthält das Acrylatcopolymer (b), bezogen auf das Gesamtgewicht der kosmetischen Zusammensetzung, z. B. in einer Menge von 0,1 bis 5,0 Gew.-%, bevorzugt 1,0 bis 4,0 Gew.-%, weiterhin bevorzugt 1,5 bis 3,0 Gew.-%, jeweils angegeben als Feststoffgehalt aktiver Substanz in der kosmetischen Zusammensetzung.

Die erfindungsgemäßen kosmetischen Zusammensetzungen zeichnen sich gegenüber alternativen kosmetischen Mitteln neben den oben genannten Vorteilen insbesondere auch durch einen verbesserten Langzeithalt aus. Als für die kosmetischen Eigenschaften der erfindungsgemäßen Mittel besonders vorteilhaft hat sich ein Gewichtsverhältnis der Polymere a) und b) in dem kosmetischen 5:1 bis 1:5, vorzugsweise 3:1 bis 1:3 und insbesondere 2:1 bis 1:2 erwiesen.

In einer besonders bevorzugten Ausführungsform der vorliegenden Erfindung enthält die kosmetische Zusammensetzung als das Vinylpyrrolidon-Copolymer (a) das im Handel unter der Bezeichnung Gafquat® HS 100 erhältliche Copolymer und als das anionische Acrylatcopolymer (b) das im Handel unter der Bezeichnung AquaStyle® SH-100 erhältliche Copolymer. Bei dieser Kombination wurden besonders gute Ergebnisse hinsichtlich einer Kombination von Steifheit und Langzeithalt erzielt. Besonders vorteilhaft ist diese Polymerkombination bei Stylingprodukten in Gelform.
Weitere allgemein geforderte Eigenschaften von Stylingprodukten, wie z. B. Feuchtebeständigkeit und niedrige Klebrigkeit, werden insbesondere mit dieser Kombination gleichfalls erzielt, besonders bei Konfektionierung als Haargel.

Die Copolymere (a) und (b) werden in der kosmetischen Zusammensetzung vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten daher 2-Amino-2-methylpropanol. Das 2-Amino-2-methylpropanol wird in den erfindungsgemäßen Mitteln vorzugsweise in einer Menge eingesetzt, welche die zur Neutralisation der Acrylatcopolymere (a) und (b) benötigte Menge nicht überschreitet. Vorzugsweise beträgt die in den erfindungsgemäßen Zusammensetzungen eingesetzte Mengen an 2-Amino-2-methylpropanol 80 bis 100%, besonders bevorzugt 90 bis 100% und insbesondere 95 bis 100% der zur vollständigen Neutralisation der Acrylatcopolymere (a) und (b) benötigten Menge. In einer bevorzugten Ausführungsform beträgt der Gewichtsanteil des 2-Amino-2-methylpropanols am Gesamtgewicht des kosmetischen Mittels 0,05 bis 7,0 Gew.-%, bevorzugt 0,1 bis 5,0 Gew.% und insbesondere 0,1 bis 3,0 Gew.-%.

Zusammenfassend enthält eine bevorzugte kosmetische Zusammensetzung zur temporären Umformung keratinischer Fasern bezogen auf ihr Gesamtgewicht:
(a) 1,0 bis 5,0 mindestens einer polymeren quartären Ammoniumverbindung aus der Gruppe der Vinylpyrrolidon Copolymere, welches zumindest aus folgenden Monomereinheiten aufgebaut ist:
   (a1) Vinylpyrrolidon
   (a2) Methacrylamidopropyltrimethylammoniumchlorid
   und
(b) 0,1 bis 5,0 mindestens eines anionischen Acrylatcopolymers (b), welches zumindest aus folgenden Monomereinheiten aufgebaut ist:
   (b1) mindestens eine Methacrylsäureeinheit
   (b2) mindestens eine Acrylsäureethylestereinheit
   (b3) mindestens eine Methacrylsäureestereinheit, die von der Acrylsäureethylestereinheit (b2) verschieden ist und eine hydrophobe Gruppe als Estergruppe aufweist.

Bevorzugt enthält die kosmetische Zusammensetzung der vorliegenden Erfindung eine oder mehrere weitere, als Verdickungsmittel oder Gelbildner wirkende Komponente(n), die von den Copolymeren (a) und (b) verschieden ist/sind und ebenfalls die Filmbildung unterstützen. Beispiele sind kationische, anionische, nichtionische oder amphotere Polymere. Der Gewichtsanteil dieser weiteren Komponente am Gesamtgewicht der kosmetischen Zusammensetzung kann aufgrund der Anwesenheit der Komponenten (a) und (b) vergleichsweise niedrig sein und beträgt beispielsweise 0,02 bis 3 Gew.-%, bevorzugt 0,05 bis 1,5 Gew.-% und noch bevorzugter 0,2 bis 0,8 Gew.-%.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Beispiele für nichtionische Polymere sind:
- Vinylpyrrolidon/Vinylester-Copolymere, wie sie beispielsweise unter dem Warenzeichen Luviskol (BASF) vertrieben werden. Luviskol VA 64 und Luviskol VA 73, jeweils Vinylpyrrolidon/VinylacetatCopolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminalund Benecel (AQUALON) vertrieben werden.

- Schellack.
- Polyvinylpyrrolidone, wie sie beispielsweise unter der Bezeichnung Luviskol (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Bevorzugt ist die weitere als Gelbildner wirkende Komponente eine Homopolyacrylsäure (INCI: Carbomer), die im Handel unter dem Namen Carbopol® in unterschiedlichen Ausführungen erhältlich ist. Das Carbomer ist bevorzugt in einem Anteil von 0,02 bis 3 Gew.-%, bevorzugt 0,05 bis 1,5 Gew.-% und noch bevorzugter 0,2 bis 0,8 Gew.-%, in Bezug auf das Gesamtgewicht der kosmetischen Zusammensetzung, enthalten.

Aufgrund ihrer kosmetischen Wirkung in Kombination mit den Copolymeren a) und b) erfindungsgemäß bevorzugt eingesetzte filmbildenden Polymere sind insbesondere die Polyvinylpyrrolidone (INCI-Bezeichnung: PVP) sowie die Vinylpyrrolidon/Vinylacetat-Copolymere (INCI-Bezeichnung VP/VA Copolymer), wobei der Gewichtsanteil dieser Polymere vorzugsweise auf Mengen zwischen 1,0 und 10 Gew.-% beschränkt wird. Besonders bevorzugte erfindungsgemäße kosmetische Zusammensetzungen sind daher dadurch gekennzeichnet, dass sie bezogen auf ihr Gesamtgewicht weiterhin 1,0 bis 10 Gew.-% Polyvinylpyrrolidon und/oder Vinylpyrrolidon/Vinylacetat-Copolymer, vorzugsweise Polyvinylpyrrolidon enthalten. Besonders bevorzugte kosmetische Mittel weisen einen Gewichtsanteil des Polyvinylpyrrolidons und/oder Vinylpyrrolidon/Vinylacetat-Copolymers c) am Gesamtgewicht des kosmetischen Mittels von 2,0 bis 8,5 Gew.-%, vorzugsweise von 3,0 bis 7,0 Gew.-% auf.

Die erfindungsgemäße kosmetische Zusammensetzung kann weitere übliche Stoffe von Stylingprodukten enthalten. Als weitere geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von 6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in der erfindungsgemäßen Zusammensetzung bevorzugt Emulgatoren bzw. oberflächenaktive Mittel enthalten. Bevorzugt sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Die erfindungsgemäßen kosmetischen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien mit vorzugsweise mindestens 10 Gew.-% Wasser, berechnet auf das Gesamtgewicht des Mittels.

Besonders bevorzugt enthält der erfindungsgemäße kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält. Ganz besonders bevorzugte kosmetische Mittel weisen bezogen auf ihre Gesamtgewicht einen Wasseranteil zwischen 50 und 95 Gew.-%, bevorzugt zwischen 60 und 90 Gew.-% und insbesondere zwischen 65 und 85 Gew.-% auf.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

### Tabellarische Übersicht

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Misc | add 100 | add 100 | add 100 | add 100 | add 100 |

| | Formel 1a | Formel 2a | Formel 3a | Formel 4a | Formel 5a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Misc | add 100 | add 100 | add 100 | add 100 | add 100 |

| | Formel 1b | Formel 2b | Formel 3b | Formel 4b | Formel 5b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Misc | add 100 | add 100 | add 100 | add 100 | add 100 |

| | Formel 6 | Formel 7 | Formel 8 | Formel 9 | Formel 10 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 6a | Formel 7a | Formel 8a | Formel 9a | Formel 10a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 6b | Formel 7b | Formel 8b | Formel 9b | Formel 10b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 11 | Formel 12 | Formel 13 | Formel 14 | Formel 15 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 11a | Formel 12a | Formel 13a | Formel 14a | Formel 15a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 11b | Formel 12b | Formel 13b | Formel 14b | Formel 15b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Misc | add 100 | add 100 | add 100 | add 100 | add 100 |

| | Formel 16 | Formel 17 | Formel 18 | Formel 19 | Formel 20 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

| | Formel 16a | Formel 17a | Formel 18a | Formel 19a | Formel 20a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

| | Formel 16b | Formel 17b | Formel 18b | Formel 19b | Formel 20b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Misc | add 100 | add 100 | add 100 | add 100 | add 100 |

| | Formel 21 | Formel 22 | Formel 23 | Formel 24 | Formel 25 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

| | Formel 21a | Formel 22a | Formel 23a | Formel 24a | Formel 25a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

| | Formel 21b | Formel 22b | Formel 23b | Formel 24b | Formel 25b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Misc | add 100 | add 100 | add 100 | add 100 | add 100 |

| | Formel 26 | Formel 27 | Formel 28 | Formel 29 | Formel 30 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 26a | Formel 27a | Formel 28a | Formel 29a | Formel 30a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 26b | Formel 27b | Formel 28b | Formel 29b | Formel 30b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 31 | Formel 32 | Formel 33 | Formel 34 | Formel 35 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 31a | Formel 32a | Formel 33a | Formel 34a | Formel 35a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 31b | Formel 32b | Formel 33b | Formel 34b | Formel 35b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Polyvinylpyrrolidon | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 36 | Formel 37 | Formel 38 | Formel 39 | Formel 40 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 36a | Formel 37a | Formel 38a | Formel 39a | Formel 40a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 36b | Formel 37b | Formel 38b | Formel 39b | Formel 40b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Vinylpyrrolidon/Vinylacetat-Copolymer | 1,0 bis 10 | 2,0 bis 8,5 | 2,0 bis 8,5 | 3,0 bis 7,0 | 3,0 bis 7,0 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 41 | Formel 42 | Formel 43 | Formel 44 | Formel 45 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 41a | Formel 42a | Formel 43a | Formel 44a | Formel 45a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 41b | Formel 42b | Formel 43b | Formel 44b | Formel 45b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| Carbomer | 0,02 bis 3,0 | 0,05 bis 2,0 | 0,05 bis 1,5 | 0,2 bis 1,0 | 0,4 bis 0,8 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 46 | Formel 47 | Formel 48 | Formel 49 | Formel 50 |
|---|---|---|---|---|---|
| Copolymer a) | 1,0 bis 5,0 | 1,5 bis 4,0 | 1,5 bis 4,0 | 2,0 bis 3,0 | 2,0 bis 3,0 |
| Copolymer b) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 46a | Formel47a | Formel48a | Formel49a | Formel 50a |
|---|---|---|---|---|---|
| Copolymer a): Polyquaternium-28 | 1,0 bis 5,0 | 1,5 bis 4,0 | 1,5 bis 4,0 | 2,0 bis 3,0 | 2,0 bis 3,0 |
| Copolymer b): Acrylates Copolymer (and) Water | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Wasser | 50 bis 95 | 50 bis 95 | 60 bis 90 | 60 bis 90 | 65 bis 85 |
| Misc | add 100 | add 100 | add 100 | add 100 | add 100 |

| | Formel 46b | Formel 47b | Formel 48b | Formel 49b | Formel 50b |
|---|---|---|---|---|---|
| Copolymer a): Gafquat® HS 100 (Angaben als Feststoffgehalt) | 1,0 bis 5,0 | 1,5 bis 4,0 | 1,5 bis 4,0 | 2,0 bis 3,0 | 2,0 bis 3,0 |
| Copolymer b): AquaStyle® SH-100 (Angaben als Feststoffgehalt) | 0,1 bis 5,0 | 0,1 bis 5,0 | 1,0 bis 4,0 | 1,0 bis 4,0 | 1,5 bis 3,0 |
| PEG-40 Hydrogenated Castor Oil | 0,05 bis 1,5 | 0,1 bis 1,0 | 0,2 bis 0,9 | 0,3 bis 0,8 | 0,4 bis 0,6 |
| Wasser | 50 bis 95 | 50 bis 95 | 60 bis 90 | 60 bis 90 | 65 bis 85 |
| Misc | add 100 | add 100 | add 100 | add 100 | add 100 |

Unter "Misc" ist erfindungsgemäß ein kosmetischer Träger zu verstehen, insbesondere (sofern nicht separat angeführt) Wasser und ggf. weitere übliche Bestandteile von Stylingprodukten.

Die kosmetische Zusammensetzung der vorliegenden Erfindung kann in den für die temporäre Umformung von Haaren üblichen Formen konfektioniert sein, z. B. als Haargel, Haarspray Haarschaum oder Haarwachs. Bevorzugt ist die Konfektionierung als Haargel.

Sowohl Haarschäume als auch Haarsprays erfordern die Anwesenheit von Treibmitteln. Erfindungsgemäß sollten dafür jedoch bevorzugt keine oder nur geringe Mengen an Kohlenwasserstoffen eingesetzt werden. Propan, Propan/Butan-Gemische und Dimethylether sind erfindungsgemäß besonders geeignete Treibmittel.

Die vorliegende Erfindung betrifft auch die Verwendung von erfindungsgemäßen kosmetischen Zusammensetzungen zur temporären Umformung von keratinischen Fasern, insbesondere von menschlichen Haaren, sowie ein Verfahren zur temporären Verformung von keratinischen Fasern, insbesondere menschlichen Haaren, bei dem die erfindungsgemäße kosmetische Zusammensetzung auf keratinische Fasern appliziert wird.

Ein weiterer Gegenstand dieser Patentanmeldung ist die Verwendung einer erfindungsgemäßen kosmetischen Zusammensetzung zur Verbesserung der Feuchtebeständigkeit temporär verformter keratinischer Fasern.

### Beispiele

Es wurde folgende Haargele hergestellt:

| **Komponente/Rohstoff** | **INCI-Bezeichnung oder chemische Bezeichnung** | **V1** | **V2** | **E1** |
|---|---|---|---|---|
| Gafquat® HS 100¹ | Polyquaternium-28 | 25 | -- | 12,5 |
| AquaStyle® SH-100 ² | Acrylates Copolymer (and) Water | -- | 16,5 | 8,25 |
| AMP-ULTRA PC 2000 | Aminomethyl Propanol | 0,4 | 0,3 | 0,3 |
| Wasser | | 74,6 | 83,2 | 78,95 |
| **Gesamt** | | 100 | 100 | 100 |

| | | | | |
|---|---|---|---|---|
| ¹ 20 Gew.-% Aktivsubstanz in Wasser ² 30 Gew.-% Aktivsubstanz in Wasser | | | | |

Die Mengenangaben in der Tabelle sind in Gew.-% des jeweiligen Rohstoffs, bezogen auf die gesamte Zusammensetzung, angegeben. Der Polymergehalt in jeder der Zusammensetzungen V1, V2 und E1 betrug 5,0 Gew.-%.

Für die erhaltenen Stylingmittel wurde mittels eines HHCR-Test (High Humidity Curl Retention-Test: 6h) an aufgereinigten Kerling-Haarsträhnen die Feuchtebeständigkeit bestimmt (Mittelwert bei Bestimmung an je 5 Haarsträhnen):

| | **V1** | **V2** | **E1** |
|---|---|---|---|
| HHCR | 49% | 72% | 91% |

Die erfindungsgemäße Polymerkombination E1 zeigte demnach einen deutlich überadditiven, synergistischen Effekt in Bezug auf die Feuchtebeständigkeit.

## Patentansprüche

1. Eine kosmetische Zusammensetzung zur temporären Umformung keratinischer Fasern, welche enthält:
(a) mindestens eine polymere quartäre Ammoniumverbindung aus der Gruppe der Vinylpyrrolidon Copolymere, welche zumindest aus den folgenden Monomereinheiten aufgebaut ist:
(a1) Vinylpyrrolidon
(a2) Methacrylamidopropyltrimethylammoniumchlorid
und
(b) mindestens ein anionisches Acrylatcopolymer (b), welches zumindest aus folgenden Monomereinheiten aufgebaut ist:
(b1) mindestens eine (Meth)Acrylsäureeinheit
(b2) mindestens eine (Meth)Acrylsäureethylestereinheit
(b3) mindestens eine (Meth)Acrylsäureestereinheit, die von der (Meth)Acrylsäureethylestereinheit (b2) verschieden ist und eine hydrophobe Gruppe als Estergruppe aufweist.

2. Kosmetische Zusammensetzung nach Anspruch 1, wobei das mindestens eine Vinylpyrrolidon-Copolymer (a) ein Copolymer von Methacrylamidopropyltrimethylammoniumchlorid (MAPTAC) mit Vinylpyrrolidon ist, welches 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthält.

3. Kosmetische Zusammensetzung nach einem der vorhergehenden Ansprüche, wobei die Zusammensetzung das Vinylpyrrolidon-Copolymer (a) in einem Anteil von 1,0 bis 5,0 Gew.-%, bevorzugt 1,5 bis 4,0 Gew.-% und insbesondere von 2,0 bis 3,0 Gew.-% enthält, bezogen auf das Gesamtgewicht der kosmetischen Zusammensetzung.

4. Kosmetische Zusammensetzung nach einem der vorhergehenden Ansprüche, wobei das anionische Acrylatcopolymer (b) Methacrylsäure als Monomereinheit (b1) und Ethylacrylat als Monomereinheit (b2) aufweist.

5. Kosmetische Zusammensetzung nach einem der vorhergehenden Ansprüche, wobei das anionische Acrylatcopolymer (b) einen (Meth)Acrylsäurealkylester als Monomereinheit (b3) aufweist.

6. Kosmetische Zusammensetzung nach einem der vorhergehenden Ansprüche, wobei die Zusammensetzung das anionische Acrylatcopolymer (b) in einem Anteil von 0,1 bis 5,0 Gew.-%,
bevorzugt 1,0 bis 4,0 Gew.-% und insbesondere von 1,5 bis 3,0 Gew.-% enthält, bezogen auf das Gesamtgewicht der kosmetischen Zusammensetzung.

7. Kosmetische Zusammensetzung einem der vorhergehenden Ansprüche, wobei die Zusammensetzung weiterhin mindestens ein von den Copolymeren (a) und (b) verschiedenes Polymer (c), insbesondere ein anionisches oder nichtionisches Polymer (c), enthält.

8. Kosmetische Zusammensetzung nach einem der vorhergehenden Ansprüche, wobei die Zusammensetzung als Haargel, Haarspray, Haarschaum oder Haarwachs, insbesondere als Haargel, vorliegt.

9. Verwendung einer kosmetischen Zusammensetzung nach einem der Ansprüche 1 bis 8 zur temporären Umformung keratinischer Fasern.

10. Verfahren zur temporären Verformung von keratinischen Fasern, insbesondere menschlichen Haaren, bei dem die kosmetische Zusammensetzung nach einem der Ansprüche 1 bis 8 auf keratinische Fasern appliziert wird.

## Claims

1. A cosmetic composition for temporarily shaping keratin fibers, containing:
(a) at least one polymeric quaternary ammonium compound from the group of vinylpyrrolidone copolymers, made up of at least the following monomer units:
(a1) vinylpyrrolidone
(a2) methacrylamidopropyltrimethylammonium chloride
and
(b) at least one anionic acrylate copolymer (b), made up of at least the following monomer units:
(b1) at least one (meth)acrylic acid unit
(b2) at least one (meth)acrylic acid ethyl ester unit
(b3) at least one (meth)acrylic acid ester unit which is different from the (meth)acrylic acid ethyl ester unit (b2) and comprises a hydrophobic group as the ester group.

2. The cosmetic composition according to claim 1, wherein the at least one vinylpyrrolidone copolymer (a) is a copolymer of methacrylamidopropyltrimethylammonium chloride (MAPTAC) with vinylpyrrolidone, which contains from 40 to 95 mol.%, preferably from 42.5 to 90 mol.%, more preferably from 45 to 85 mol.%, and in particular from 50 to 80 mol.%, of vinylpyrrolidone.

3. The cosmetic composition according to one of the preceding claims, wherein the composition contains the vinylpyrrolidone copolymer (a) in a proportion of from 1.0 to 5.0 wt.%, preferably from 1.5 to 4.0 wt.%, and in particular from 2.0 to 3.0 wt.%, based on the total weight of the cosmetic composition.

4. The cosmetic composition according to one of the preceding claims, wherein the anionic acrylate copolymer (b) comprises methacrylic acid as monomer unit (b1) and ethyl acrylate as monomer unit (b2).

5. The cosmetic composition according to one of the preceding claims, wherein the anionic acrylate copolymer (b) comprises a (meth)acrylic acid alkyl ester as monomer unit (b3).

6. The cosmetic composition according to one of the preceding claims, wherein the composition contains the anionic acrylate copolymer (b) in a proportion of from 0.1 to 5.0 wt.%, preferably from 1.0 to 4.0 wt.%, and in particular from 1.5 to 3.0 wt.%, based on the total weight of the cosmetic composition.

7. The cosmetic composition according to one of the preceding claims, wherein the composition further contains at least one polymer (c) which is different from copolymers (a) and (b), in particular an anionic or nonionic polymer (c).

8. The cosmetic composition according to one of the preceding claims, wherein the composition is present as a hair gel, hair spray, hair foam or hair wax, in particular as a hair gel.

9. The use of a cosmetic composition according to one of claims 1 to 8 for temporarily shaping keratin fibers.

10. A method for temporarily shaping keratin fibers, in particular human hair, wherein the cosmetic composition according to one of claims 1 to 8 is applied to keratin fibers.

## Revendications

1. Composition cosmétique destinée à la mise en forme temporaire de fibres kératiniques, laquelle composition contient :
(a) au moins un composé d'ammonium quaternaire polymère du groupe des copolymères de la vinylpyrrolidone, lequel composé est au moins constitué des motifs monomère suivants :
(a1) la vinylpyrrolidone
(a2) le chlorure de méthacrylamidopropyltriméthylammonium et
(b) au moins un copolymère d'acrylate anionique (b), lequel est constitué au moins des motifs monomère suivants :
(b1) au moins un motif acide (méth)acrylique
(b2) au moins un motif ester éthylique d'acide (méth)acrylique
(b3) au moins un motif ester d'acide (méth)acrylique différent du motif ester éthylique d'acide (méth)acrylique (b2) et comportant un groupe hydrophobe en tant que groupe ester.

2. Composition cosmétique selon la revendication 1, dans laquelle l'au moins un copolymère de la vinylpyrrolidone (a) est un copolymère du chlorure de méthacrylamidopropyltriméthylammonium (MAPTAC) avec de la vinylpyrrolidone, lequel contient de 40 à 95 % en mole, de préférence de 42,5 à 90 % en mole, plus préférablement de 45 à 85 % en mole et en particulier de 50 à 80 % en mole de vinylpyrrolidone.

3. Composition cosmétique selon l'une des revendications précédentes, la composition contenant le copolymère de vinylpyrrolidone (a) dans une proportion de 1,0 à 5,0 % en poids, de manière préférée de 1,5 à 4,0 % en poids et en particulier de 2,0 à 3,0 % en poids, rapportée au poids total de la composition cosmétique.

4. Composition cosmétique selon l'une des revendications précédentes, le copolymère d'acrylate anionique (b) comportant de l'acide méthacrylique comme motif monomère (b1) et de l'acrylate d'éthyle comme motif monomère (b2).

5. Composition cosmétique selon l'une des revendications précédentes, le copolymère d'acrylate anionique (b) comportant un ester alkylique d'acide (méth)acrylique comme motif monomère (b3).

6. Composition cosmétique selon l'une des revendications précédentes, la composition contenant le copolymère d'acrylate anionique (b) dans une proportion allant de 0,1 à 5,0 % en poids, de manière préférée de 1,0 à 4,0 % en poids et en particulier de 1,5 à 3,0 % en poids, rapportée au poids total de la composition cosmétique.

7. Composition cosmétique selon l'une des revendications précédentes, la composition contenant en outre au moins un polymère (c) différent des copolymères (a) et (b), en particulier un polymère (c) anionique ou non ionique.

8. Composition cosmétique selon l'une des revendications précédentes, la composition se présentant sous forme de gel capillaire, de spray capillaire, de mousse capillaire ou de cire capillaire, en particulier sous forme de gel capillaire.

9. Utilisation d'une composition cosmétique selon l'une des revendications 1 à 8 pour la mise en forme temporaire de fibres kératiniques.

10. Procédé de mise en forme temporaire de fibres kératiniques, en particulier de cheveux, lors duquel la composition cosmétique selon l'une des revendications 1 à 8 est appliquée sur les fibres kératiniques.
